# EUROPÄISCHE PATENTSCHRIFT

(11) **EP 1 112 049 B1**
(45) Veröffentlichungstag und Bekanntmachung des Hinweises auf die Patenterteilung: **26.11.2003**
(21) Anmeldenummer: 99947307.7
(22) Anmeldetag: 10.09.1999
(51) Int. Cl.: A61F 2/40

(54) **IMPLANTIERBARE PROTHESE MIT ZUMINDEST ZWEI GEGENEINANDER VERSTELLBAREN ABSCHNITTEN SOWIE VERWENDUNG VERSTELLBARER ABSCHNITTE**
IMPLANTABLE PROSTHESIS HAVING AT LEAST TWO SECTIONS WHICH CAN BE DISPLACED IN RELATION TO ONE ANOTHER, AND THE USE OF DISPLACEABLE SECTIONS
PROTHESE IMPLANTABLE COMPORTANT AU MOINS DEUX SECTIONS DEPLA ABLES L'UNE PAR RAPPORT A L'AUTRE ET UTILISATION DES SECTIONS DEPLACABLES

(30) Priorität: 11.09.1998 DE 19841612
(43) Veröffentlichungstag der Anmeldung: 04.07.2001
(73) Patentinhaber: Argomedical AG, 6330 Cham (CH)
(72) Erfinder: KROPF, Philipp, CH-8142 Uitikon (CH)
(74) Vertreter: Hiebsch, Gerhard F., Dipl.-Ing.
(86) Internationale Anmeldenummer: EP9906689
(87) Internationale Veröffentlichungsnummer: WO00015154

(56) Entgegenhaltungen:
- EP-A- 0 024 442
- EP-A- 0 599 429
- EP-A- 0 679 375
- EP-A- 0 712 617
- EP-A- 0 903 128
- WO-A-98/46172
- FR-A- 2 721 200

## Beschreibung

Die Erfindung betrifft eine implantierbare Prothese mit zumindest zwei gegeneinander verstellbaren Abschnitten--insbesondere eine Prothese für das obere Ende eines menschlichen Oberarmes -- mit in einen Knockenkanal einführbarem Schaft und einem der benachbarten Gelenkpfanne zuzuordnenden kalottenförmigen Gelenkkopf, der mittels einer -- sowohl zu ihm als auch zum Schaft jeweils einen in eine Bohrung einsetzbaren Steckbolzen od.dgl. zapfenartige Anformung aufweisenden -- Zwischenscheibe kreisförmigen Umfanges an eine Stirnfläche des Schaftes in exzentrischer Lage -- zu deren Mittelachse -- anschließbar ist. Zudem erfasst die Erfindung die Verwendung verstellbarer Abschnitte und einer Zwischenscheibe an einer Verstellebene.

Eine derartige Prothese ist der EP-A-0 599 429 zu entnehmen; letztere beschreibt das Femoralteil einer Hüftgelenk-Endoprothese, der einen Schaft, einen kugelförmigen Kopf und ein den Schaft mit dem Kopf verbindendes Verbindungsteil aufweist. Am proximalen Ende des Schafts ist eine konische Vertiefung ausgebildet, bei der ein erster konischer Zapfen am schaftnahen Ende des Verbindungsteils zugeordnet sowie ein zweiter konischer Zapfen am kopfnahen Ende des Verbindungsteils angeordnet ist, der sich in eine entsprechende konische Vertiefung im Kopf erstreckt. Die Längsachse des zweiten konischen Zapfens bietet eine Parallelversetzung bezüglich der Längsachse des ersten konischen Zapfens an. Jenes Verbindungsteil enthält Befestigungsvorrichtungen, um das Verbindungsteil bezüglich des Schafts zu fixieren und undrehbar werden zu lassen. Zumindest eine Befestigung ist dem Verbindungsteil zugeordnet, die sich in einer vorgegebenen Relativposition des Schafts und des Verbindungsteils formschlüssig in eine entsprechende Vertiefung erstreckt.

By einer Gelenkprothese nach EP 0 549 480 A1 ragt von der von den Oberflächen der diametral keilförmigen Zwischenscheibe jeweils ein zentrischer Steckzapfen ab. Für den kopfseitigen Steckzapfen ist im Gelenkkopf eine exzentrische Bohrung vorgesehen und um jene ein Kranz kleinerer Bohrungen zur Aufnahme eines sowohl in diese als auch in die Zwischenscheibe eingreifenden achsparallelen Fixierstiftes. Das Verstellen des Gelenk- oder Humeruskopfes erfolgt stufenweise, kann also nicht in allen Situationen den Gegebenheiten angepaßt werden. Zudem erlaubt es die Konstruktion dieser Schultergelenkprothese nicht, Schnittachsen bzw. Resektionsachsen zu verschieben.

Die EP 0 712 617 A1 beschreibt ebenfalls eine Gelenkprothese, in deren Schaft der Gelenkkopf mittels eines Kugelgelenkes lagert. Auch dieses bietet keine Feinabstimmung des Gelenkkopfes an.

In Kenntnis dieses Standes der Technik hat sich der Erfinder das Ziel gesetzt, eine Prothese der eingangs erwähnten Art so auszugestalten, dass bei ihrer Anpassung an die jeweils anatomischen Gegebenheiten der mit ihr zu verbindenden menschlichen Knochen problemlos auch eine Feinsteinstellung möglich ist.

Zur Lösung dieser Aufgabe führt die Lehre des unabhängigen Anspruches; die Unteransprüche geben günstige Weiterbildungen an.

Erfindungsgemäß sind die gegeneinander verstellbaren Abschnitte der Prothese an zumindest einer von den Oberfläche eines Abschnitts bestimmten Verstellebene gegeneinander um Achsen stufenlos verstellbar angeordnet, welche von den Steckbolzen der Zwischenscheibe bestimmt sind. Bevorzugt soll einer der Steckbolzen in radialem Abstand zur Achse der Zwischenscheibe angeordnet sein.

Dank dieser Maßgaben können Schaft und Gelenkkopf -- oder zwei entsprechend zu verstellende Teile anderer Implantatprothesen -- an jener Stirnfläche oder parallel dazu stufenlos gegeneinander verschoben werden.

Nach einem anderen Merkmal der Erfindung soll die -- in eine ihrer Form gleichende Ausnehmung eines der benachbarten Abschnitte einsetzbare -- Zwischenscheibe mit einem zentrischen Steckbolzen in eine entsprechende sowie exzentrisch in eine Anschlussebene des Gelenkkopfes eingeformte Ausnehmung eingesetzt werden; der andere -- exzentrisch angebrachte -- Steckbolzen ist der Stirnfläche des Schaftes zugeordnet. Jene Anschlussebene bestimmt im übrigen eine von zwei Verstellebenen der Prothese; die andere liegt in der Schaftstirnfläche.

Als günstig hat es sich erwiesen, den Durchmesser der Zwischenscheibe etwa dem halben Durchmesser des Gelenkkopfes entsprechen zu lassen sowie ihre Höhe etwa der jeweiligen Höhe ihrer Steckbolzen; die Höhe der Zwischenscheibe soll dazu etwa einem Drittel ihres Durchmessers gleichen bei einem bevorzugten Bolzendurchmesser, dessen Länge sich zum Durchmesser seiner Zwischenscheibe verhält wie etwa 1 : 2,5 bis 1 : 3,0. Zudem soll der radiale Abstand des exzentrisch vorgesehenen Steckbolzens von der Achse der Zwischenscheibe etwa dem halben Bolzendurchmesser entsprechen.

Es wird deutlich, dass die Anschlussfläche des erfindungsgemäßen Gelenkkopfes eine aus ihrer Achse verschobene flache Sackausnehmung zum Einsetzen der auf beiden Scheibenseiten mit einem Steckbolzen ausgestatteten Zwischenscheibe anbietet. Deren beide Steckbolzen sind radial gegeneinander versetzt und erlauben es, den Gelenkkopf gegenüber dem Prothesenschaft stufenlos zu verschieben.

Bevorzugt werden die Steckbolzen im Presssitz in ihre Sackbohrung eingebracht. Sie können beispielsweise jeweils einen ringförmigen Presssitzbereich anbieten, dessen Höhe mit einem Zehntel der Bolzenhöhe ausreichend bemessen ist, jedoch auch breiter sein kann.

In einer weiteren Ausgestaltung mag der Steckbolzen leicht konisch ausgeführt sein; diese Konizität des Steckbolzens gewährleistet ebenfalls dessen Sitz in der zylindrischen Sackbohrung.

Die Lage der Zwischenscheibe wird nach einem weiteren Merkmal der Erfindung durch deren Zusammenstecken mit dem Gelenkkopf bzw. dem Schaft fixiert.

In einer spezifischen Ausführung der Erfindung kann der Durchmesser der Steckbolzen 1 bis 9 mm messen, bevorzugt bis 7 mm. Dazu weist dann die Zwischen- oder Exzenterscheibe einen Durchmesser von 10 bis 35 mm -- vorzugsweise 12 bis 30 mm -- auf sowie eine Dicke von 2 bis 8 mm, vorzugsweise von 3 bis 7 mm.

Bei derartigen Maßen hat es sich als günstig erwiesen, wenn die flache Ausnehmung für die Zwischenscheibe im kalottenartigen Gelenkkopf der implantierbaren Prothese aus dessen Mittelachse um bis zu 10 mm -- vorzugsweise bis zu 8 mm -verschoben liegt.

Die erfindungsgemäße Zuordnung zweier verstellbarer Abschnitte und ihrer Zwischenscheibe an einer Verstellebene kann auch für implantierbare Prothesen anderer Art Verwendung finden.

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
- Fig. 1:: eine schematische Seitenansicht einer modularen Schultergelenkprothese mit einem Schaft zugeordneter Zwischenscheibe und auf diese aufsetzbarem Gelenkkopf in voneinander getrenntem Zustand;
- Fig. 2:: den einends eine Stirnfläche aufweisenden Schaft der Fig. 1;
- Fig. 3:: die Draufsicht auf die Stirnfläche des Schaftes;
- Fig. 4:: einen Querschnitt durch Fig. 2 nach deren Linie IV-IV;
- Fig. 5:: die teilweise geschnittene Frontansicht des Schaftes;
- Fig. 6:: eine Seitenansicht der Zwischenscheibe;
- Fig. 7, 8:: Draufsichten auf die Zwischenscheibe;
- Fig. 9:: einen vergrößerten Ausschnitt aus Fig. 6 nach deren Pfeil IX;
- Fig. 10:: die Draufsicht auf den Gelenkkopf;
- Fig. 11:: eine geschnittene Seitenansicht des Gelenkkopfes;
- Fig. 12:: eine gegenüber Fig. 10 vergrößerte Darstellung eines weiteren Gelenkkopfes;
- Fig. 13:: eine Frontansicht der Schultergelenkprothese in einer ersten Einstellung;
- Fig. 14 bis 17:: die Schultergelenkprothese der Fig. 13 in anderen -- zueinander unterschiedlichen -- Einstellungen ihres Gelenkkopfes.

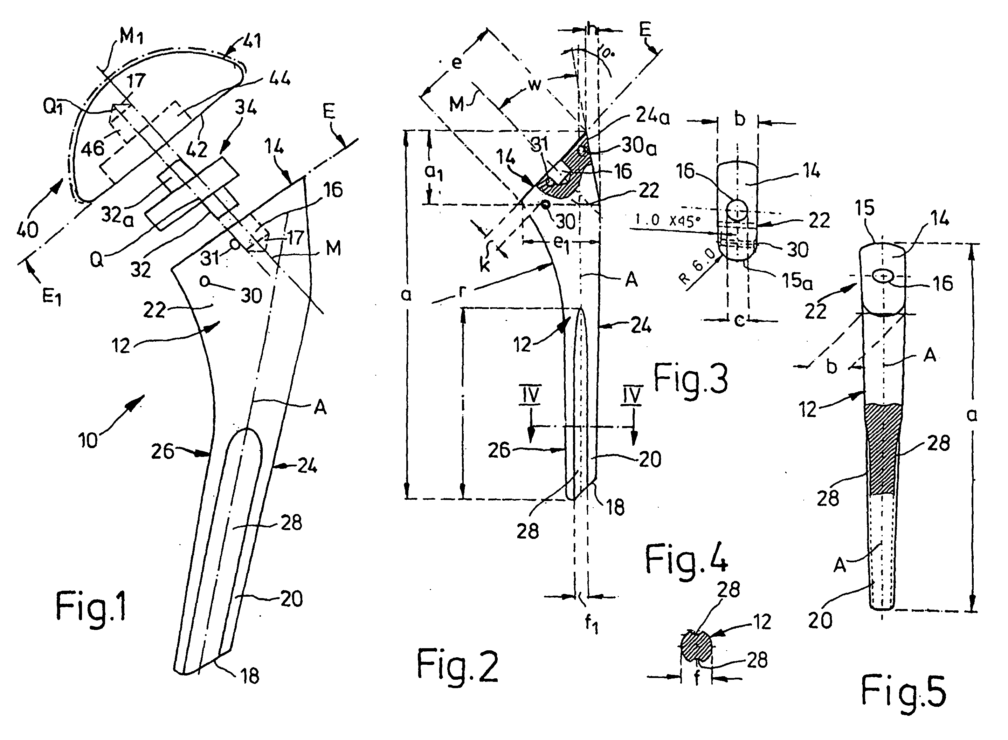

Eine Prothese 10 für ein nicht dargestelltes menschliches Schultergelenk weist einen Schaft 12 mit einer Stirnfläche 14 auf, deren Mittelachse M gegen die Längsachse A des Schaftes 12 in einem Winkel w von 45° geneigt ist. Die größte Länge a dieses Schaftes 12 misst 125 mm, die Breite b der Stirnfläche 14 etwa 14 mm und deren Länge e etwa 23 mm. Die in Fig. 2 erkennbare Projektionslänge e₁ der Stirnfläche 14 in eine die Längsachse A rechtwinkelig querende Ebene beträgt dann 27,3 mm.

Die Mittelachse M der Stirnfläche 14 bestimmt in dieser die Lage eines Sackloches 16 des Durchmessers d von etwa 7 mm mit Kegelende 17. Gegenüber dem -- an einer geneigten Fußfläche 18 endenden -- freien Schaftende 20 der Breite f von 11 mm ist der Kopfbereich 22 des Schaftes 14 auf jene Länge e bzw. e₁ erweitert. Die obere, leicht gerundete Schmalkante 15 der Stirnfläche 14 ist gegenüber der Flucht ihrer Schaftseite 24 dank eines in letzterer vorgesehenen Neigungsbereiches 24ₐ der achsparallelen Länge a₁ von etwa 25 mm um ein Maß h von etwa 4,5 mm versetzt; die andere Schaftseite 26 ist zur zweiten Schmalkante 15ₐ der Stirnfläche 14 hin in einem Radius r = 70 mm auswärts gekrümmt. Im querschnittlich etwa gleichbleibenden Schaftbereich verlaufen beidseits mittige Flachnuten 28 der Breite f₁ von hier 4,6 mm sowie der Länge i von 65 mm.

In einem Abstand k von etwa 5,5 mm quert jenen Kopfbereich 22 des Schaftes 12 nahe der im Radius r gekrümmten Schaftseite 26 eine Querbohrung 30; bei 30ₐ ist in Fig. 2 -ebenfalls in Abstand zum Sackloch 16 -- eine mögliche weitere Querbohrung nahe der oberen Schmalkante 15 der Stirnfläche 14 angedeutet. Mit 31 ist eine Tangentialbohrung an das Sackloch 16 für eine den Bolzensitz fixierende Schraube bezeichnet.

Das Sackloch 16 der Stirnfläche 14 dient der Aufnahme eines Steckzapfens oder Steckbolzens 32 -- entsprechend runden Querschnitts des Durchmessers c₁ von 7 mm -- einer Zwischen- oder Exzenterscheibe 34 des Durchmessers d von 20 mm sowie der Höhe n von 5 mm, die beidseits mit einem solchen Steckbolzen 32, 32ₐ gleicher Höhe n versehen ist; der schaftwärtige Steckbolzen 32 ist in einem Achsabstand q von 3 mm zur Mittelachse Q der Exzenterscheibe 34 radial versetzt, der zweite Steckbolzen 32ₐ ragt von der Scheibenfläche 35 axial ab. Der Abstand q kann je nach Ausführung von 1 mm bis zu 10 mm -- bevorzugt bis zu 8 mm -- messen.

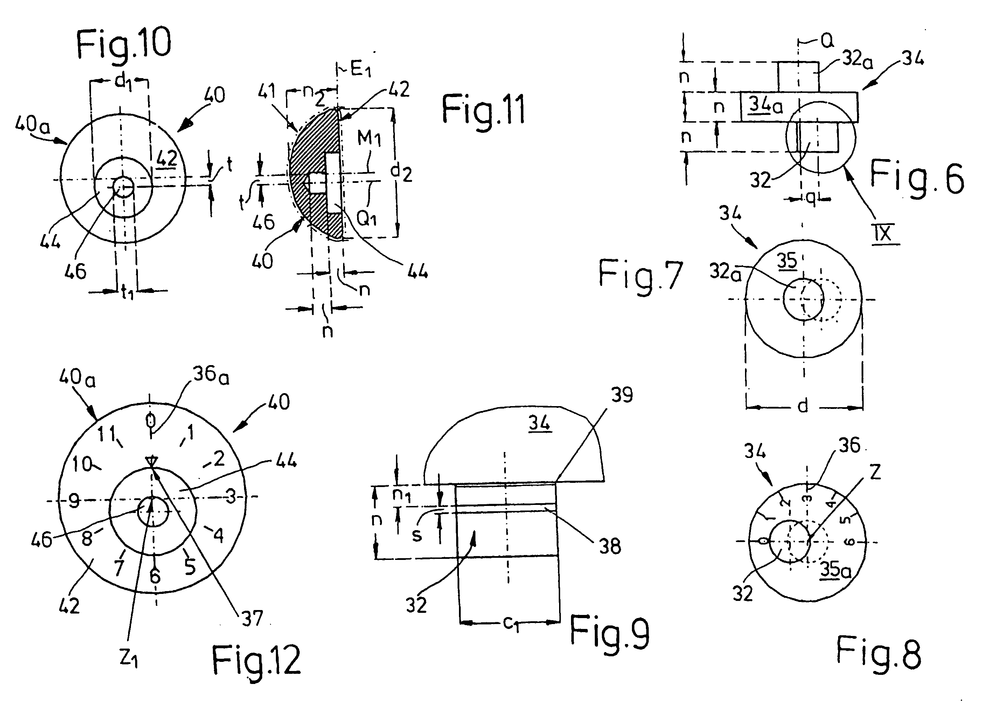

Fig. 8 verdeutlicht eine zum Scheibenzentrum Z orientierte Skala 36 mit Markierungsstrichen auf der Scheibenfläche 35ₐ, Fig. 9 den exzentrischen Steckzapfen 32 mit einem ringförmigen Presssitzbereich 38 der Breite s von 0,5 mm, der in einem Abstand n₁ von 1, 5 mm von der am Steckbolzen 32 oberhalb eines Freistiches 39 ansetzenden anderen Scheibenfläche 35ₐ der Exzenterscheibe 34. Letztere wird in zwei Varianten eingesetzt, nämlich zum einen mit dem dargestellten Presssitz für beide sich endwärts -- nicht dargestellt -- ein wenig konisch verjüngenden Steckzapfen 32, 32ₐ oder zum anderen gänzlich ohne Presssitz. Die oben erwähnte Querbohrung 30 kann eine nicht erkennbare Stiftschraube zur Erhöhung der Klemmkraft des Schaftes 12 aufnehmen.

Der zentrische Steckbolzen 32ₐ ist zum Anschluss an den kalottenartigen Humerus- oder Gelenkkopf 40 bestimmt, der an einer Anschlussfläche 42 für die Exzenterscheibe 34 eine deren Form entsprechende Ausnehmung 44 des Durchmessers d₁ mit zentrischer Sackbohrung 46 für den Steckzapfen 32ₐ anbietet. Die Bohrungsachse Q₁ liegt hier in Achsabstand t von 3 mm zur Mittelachse M₁ des Gelenkkopfes 40, und die Mittelachse M₁ der Exzenterscheibe 34 fällt in die Zylinderwand der exzentrischen Bohrung 46 der Ausnehmung 44. Auch hier kann der Achsabstand t je nach Ausführung entsprechend von 1 mm bis zu 10 mm betragen, bevorzugt bis zu 8 mm.

Die Höhe n₂ der eine polierte Oberfläche 41 aufweisenden Kalotte des Gelenkkopfes 40 ist bevorzugt proportional zur Durchmesserlänge der Teilkugelfläche ausgebildet und misst beispielsweise etwas mehr als 17 mm, der Durchmesser d₂ des Gelenkkopfes 40 hier etwas mehr als 44 mm.

Auch jene Anschlussfläche 42 bietet nach Fig. 12 eine auf ihr Zentrum Z₁ orientierte Skala 36ₐ an, zudem am Rande der Ausnehmung 44 ein Markierungsdreieck 37.

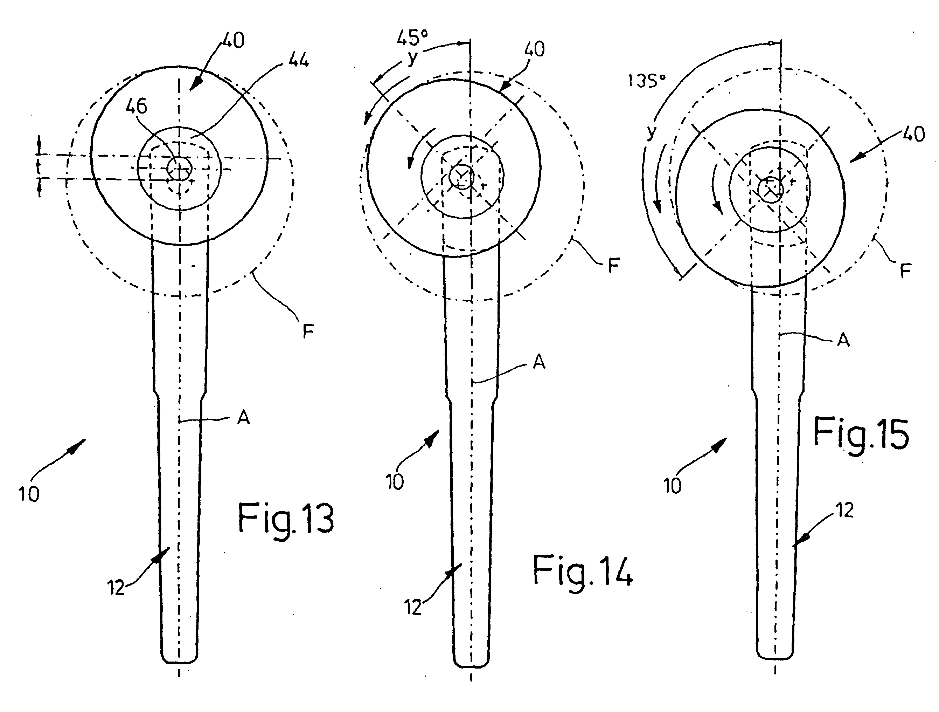

Die Fig. 13 bis 17 lassen die unterschiedlichen Einstellungen des Gelenkkopfes 40 am Schaft 12 dank der durch die Exzenterscheibe 34 mögliche Exzentrizität deutlich werden, wobei mit E und E₁ die von der Stirnfläche 14 des Schaftes 12 bzw. von der Anschlussebene 42 des Gelenkkopfes 40 bestimmten -- in Einbaulage aneinanderliegenden -- Verstellebenen sowie mit F und F₁ Umlaufkreise für die Kontur 40ₐ des Gelenkkopfes 40 bzw. die Kontur 34ₐ der Exzenterscheibe 34 bezeichnet sind.

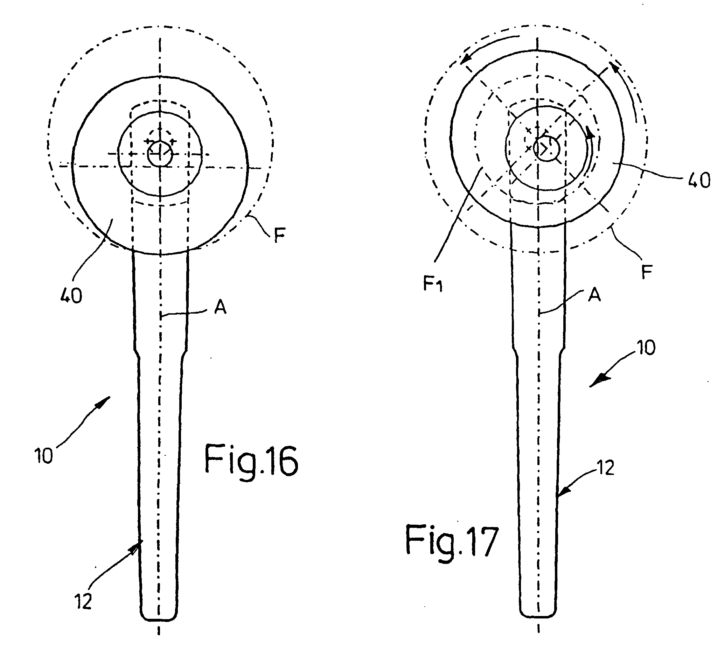

Die maximale Exzentrizität gegen außen läßt Fig. 13 mit hier t = 6 mm erkennen. Bei einer gleichzeitigen Drehung um einen Verstellwinkel y um 45° nach Fig. 14 und um 135° nach Fig. 15 von Exzenterscheibe 34 und Gelenkkopf 40 werden Positionen am Umlaufkreis F -- bei einfacher Exzentrizität -besetzt. Die maximale Exzentrizität gegen innen wird gemäß Fig. 16 nach einem Verstellwinkel von 180° erreicht.

Durch die Möglichkeit, die Exzenterscheibe 34 unabhängig vom Gelenkkopf 40 stufenlos zu drehen, kann eine unbeschränkte Vielzahl von Positionen angefahren werden. Es vermag jede Lage innerhalb der beiden Umlaufkreise F, F₁ der Fig. 17 eingestellt zu werden; in dieser Figur ist die völlige Nullstellung zu erkennen, d.h. der Gelenkkopf 40 liegt konzentrisch in den Umlaufkreisen E, E₁. In Fig. 13 und 16 bildet die Längsachse A des Schaftes 12 eine Symmetriegerade.

## Patentansprüche

1. Implantierbare Prothese mit zumindest zwei gegeneinander verstellbaren Abschnitten, insbesondere Prothese für das obere Ende eines menschlichen Oberarmes, mit in einen Knochenkanal einführbarem Schaft (12) und einem der benachbarten Gelenkpfanne zuzuordnenden kalottenförmigen Gelenkkopf (40), der mittels einer sowohl zu ihm als auch zum Schaft jeweils einen in eine Bohrung (16, 46) einsetzbaren Steckbolzen (32ₐ, 32) oder zapfenartige Anformung aufweisenden Zwischenscheibe (34) kreisförmigen Umfanges (34ₐ) an eine Stirnfläche (14) des Schaftes in exzentrischer Lage zu deren Mittelachse (M) anschließbar ist,
**dadurch gekennzeichnet,**
**dass** die gegeneinander verstellbaren Abschnitte (12, 34 bzw. 12, 40) an wenigstens einer von der Stirnfläche (14 bzw. 42) eines der Abschnitte bestimmten Verstellebene (E, E₁) gegeneinander um die zwei Achsen (M, Q bzw. Q₁) stufenlos verstellbar angeordnet sind, welche von den Steckbolzen (32, 32ₐ) der Zwischenscheibe (34) bestimmt sind.

2. Prothese nach Anspruch 1, **dadurch gekennzeichnet, dass** wenigstens einer der Steckbolzen (32) in radialem Abstand (q) zur Achse (Q) der Zwischenscheibe (34) angeordnet ist.

3. Prothese, deren Zwischenscheibe in eine ihr etwa entsprechende Ausnehmung (44) eines der benachbarten Abschnitte (14, 42) einsetzbar ist, nach Anspruch 1 oder 2, **dadurch gekennzeichnet, dass** die Zwischenscheibe (34) mit einem zentrischen Steckbolzen (32ₐ) in eine ihrer Formgebung entsprechende sowie exzentrisch in eine Anschlussfläche (42) des Gelenkkopfes (40) eingeformte Ausnehmung (44, 46) eingesetzt und der andere, exzentrisch angebrachte Steckbolzen (32) der zur Anschlussfläche, die eine Verstellebene (E₁) der Prothese (10) bestimmt, parallelen Stirnfläche (14) des Schaftes (12) zugeordnet ist.

4. Prothese nach Anspruch 2 oder 3, **dadurch gekennzeichnet, dass** der Durchmesser (d) der Zwischenscheibe (34) etwa dem halben Durchmesser (d₂) des Gelenkkopfes (40) entspricht sowie ihre Höhe (n) etwa der jeweiligen Höhe ihrer Steckbolzen (32, 32ₐ), wobei beverzugt die Höhe (n) der Zwischenscheibe (34) etwa einem Drittel ihres Durchmessers (d) entspricht.

5. Prothese nach Anspruch 4, **gekennzeichnet durch** einen Bolzendurchmesser (c₁), dessen Länge sich zum Durchmesser (d) seiner Zwischenscheibe (34) verhält wie etwa 1 : 2, 5 bis 1 : 3,0.

6. Prothese nach einem der Ansprüche 2 bis 5, **dadurch gekennzeichnet, dass** der radiale Abstand (q) des exzentrisch vorgesehenen Steckbolzens (32) von der Achse (Q) der Zwischenscheibe (34) etwa dem halben Bolzendurchmesser (c₁) entspricht.

7. Prothese nach einem der Ansprüche 1 bis 6, **dadurch gekennzeichnet, dass** der Steckbolzen (32, 32ₐ) einen ringförmigen Presssitzbereich (38) aufweist, dessen Höhe (s) bevorzugt etwa einem Zehntel bis drei Zehntel der Bolzenhöhe (n) entspricht.

8. Prothese nach einem der Ansprüche 1 bis 7, **dadurch gekennzeichnet, dass** der Steckbolzen (32, 32ₐ) für die zylindrische Sackbohrung (16, 46) sich von der Zwischenscheibe weg konisch verjüngt.

9. Prothese nach einem der Ansprüche 1 bis 8, **dadurch gekennzeichnet, dass** der Durchmesser des Steckbolzens (32, 32ₐ) 1 bis 9 mm misst, bevorzugt 3 bis 7 mm.

10. Prothese nach einem der Ansprüche 1 bis 9, **gekennzeichnet durch** eine Tangentialbohrung (31) für eine Tangentialschraube an der Sackbohrung (16, 46).

11. Prothese nach einem der Ansprüche 1 bis 10, **dadurch gekennzeichnet, dass** die Zwischenscheibe (34) einen Durchmesser (d) von 10 bis 35 mm, bevorzugt 12 bis 30 mm, aufweist.

12. Prothese nach einem der Ansprüche 1 bis 11, **dadurch gekennzeichnet, dass** die Zwischenscheibe (34) eine Dicke (n) von 2 bis 8 mm, bevorzugt von 3 bis 7 mm, aufweist.

13. Prothese nach einem der Ansprüche 1 bis 12, **dadurch gekennzeichnet, dass** die flache Ausnehmung (44) für die Zwischenscheibe (34) im Gelenkkopf (40) aus dessen Mittelachse (M₁) bis zu 10 mm verschoben ist, bevorzugt bis zu 8 mm.

14. Prothese nach einem der Ansprüche 1 bis 13, **dadurch gekennzeichnet, dass** die Scheibenfläche (35) der Zwischenscheibe (34) und/oder die Anschlussfläche (42) des Gelenkkopfes (40) mit einer der Drehrichtung zugeordneten Skala (36, 36ₐ) versehen sind/ist.

15. Verwendung verstellbarer Abschnitte (12, 40) und einer Zwischenscheibe (34) an einer Verstellebene (E, E₁) nach wenigstens einem der Ansprüche 1 bis 14 für eine Ellbogen-, Knie- oder Hüftgelenkprothese oder für eine Hand- oder Fußgelenkprothese.

16. Verwendung der Zuordnung von verstellbaren Abschnitten (12, 40) und einer Zwischenscheibe (34) an einer Verstellebene (E, E₁) nach wenigstens einem der Ansprüche 1 bis 14 für eine Wirbelsäulendiskussprothese.

## Claims

1. Implantable prosthesis having at least two sections adjustable relative to one another, in particular a prosthesis for the upper end of a human upper arm, having a shaft (12) introduceable into a bone channel and a cap-shaped joint ball (40) to be allocated to the neighbouring joint socket which ball by means of an intermediate disk (34) of circular perimeter (34ₐ) possessing relative to both itself and the shaft a socket pin (32ₐ, 32) or peg-like attachment insertable in each case into a bore (16, 46) is connectable to an end face (14) of the shaft in an eccentric position relative to the central axis (M) thereof,
**characterised in that**
at least at one adjusting plane (E, E₁) determined by the end face (14 or 42) of one of the sections, the sections (12, 34 or 12, 40) that are adjustable relative to one another are arranged to be continuously adjustable relative to one another about the two axes (M; Q or Q₁) determined by the socket pins (32, 32ₐ) of the intermediate disk (34).

2. Prosthesis according to Claim 1, **characterised in that** at least one of the socket pins (32) is arranged at a radial distance (q) from the axis (Q) of the intermediate disk (34).

3. Prosthesis, whose intermediate disk is insertable into a recess (44) corresponding approximately thereto in one of the neighbouring sections (14, 42), according to Claim 1 or 2, **characterised in that** the intermediate disk (34) is inserted by a central socket pin (32ₐ) into a recess (44, 46) matching the shape thereof and formed eccentrically in a connecting surface (42) of the joint ball (40) and the other eccentrically fitted socket pin (32) is assigned to the end face (14) parallel to the connecting surface determining an adjustment plane (E₁) of the prosthesis (10).

4. Prosthesis according to Claim 2 or 3, **characterised in that** the diameter (d) of the intermediate disk (34) is approximately half the diameter (d₂) of the joint ball (40) and the height (n) thereof is approximately the height in question of the socket pins (32, 32a) thereof, wherein preferably the height (n) of the intermediate disk (34) is approximately one third of the diameter (d) thereof.

5. Prosthesis according to Claim 4, **characterised by** a pin diameter (c₁) whose length is related to the diameter (d) of the associated intermediate disk (34) in the ratio of approximately 1 : 2.5 to 1 : 3.0.

6. Prosthesis according to one of Claims 2 to 5, **characterised in that** the radial distance (q) of the socket pin (32) provided eccentrically from the axis (Q) of the intermediate disk (34) is approximately half the pin diameter (c₁).

7. Prosthesis according to one of Claims 1 to 6, **characterised in that** the socket pin (32, 32ₐ) has an annular press-fit region (38) whose height (s) is preferably approximately one tenth to three tenths of the pin height (n).

8. Prosthesis according to one of Claims 1 to 7, **characterised in that** the socket pin (32, 32ₐ) for the cylindrical pocket bore (16, 46) tapers conically in the direction away from the intermediate disk.

9. Prosthesis according to one of Claims 1 to 8, **characterised in that** the diameter of the socket pin (32, 32ₐ) is 1 to 9 mm, preferably 3 to 7 mm.

10. Prosthesis according to one of Claims 1 to 9, **characterised by** a tangential bore (31) for a tangential screw at the pocket bore (16, 46) .

11. Prosthesis according to one of Claims 1 to 10, **characterised in that** the intermediate disk (34) has a diameter (d) of 10 to 35 mm, preferably 12 to 30 mm.

12. Prosthesis according to one of Claims 1 to 11, **characterised in that** the intermediate disk (34) has a thickness (n) of 2 to 8, preferably of 3 to 7 mm.

13. Prosthesis according to one of Claims 1 to 12, **characterised in that** the flat recess (44) for the intermediate disk (34) in the joint ball (40) is displaced from the central axis (M₁) thereof by up to 10 mm, preferably up to 8 mm.

14. Prosthesis according to one of Claims 1 to 13, **characterised in that** the disk surface (35) of the intermediate disk (34) and/or the connecting surface (42) of the joint ball (40) is/are provided with a scale (36, 36ₐ) assigned to the direction of rotation.

15. Use of adjustable sections (12, 40) and an intermediate disk (34) at an adjusting plane (E, E₁) according to at least one of Claims 1 to 14 for an elbow, knee or hip joint prosthesis or for a hand or foot joint prosthesis.

16. Use of the assignment of adjustable sections (12, 40) and an intermediate disk (34) on an adjusting plane (E, E₁) according to at least one of Claims 1 to 14 for a spinal disk prosthesis.

## Revendications

1. Prothèse implantable avec au moins deux sections réglables l'une par rapport à l'autre, tout particulièrement une prothèse pour l'extrémité supérieure d'un bras humain, avec une tige (12) insérable dans un canal osseux et une tête articulée (40) en forme de calotte, à adapter à la cavité glénoïde voisine qui, au moyen d'une poulie intermédiaire (34) de périphérie circulaire (34a), peut être connectée à une surface frontale (14) de la tige en position excentrique par rapport à son axe central (M), laquelle poulie intermédiaire comporte un axe embrochable (32a, 32), ou d'une forme similaire à un tenon, insérable dans un alésage (16, 46), aussi bien dans la tête que dans la tige
**caractérisée en ce que**
les sections réglables l'une par rapport à l'autre (12, 34 ou 12, 40) sont adaptées et réglables progressivement par rapport aux deux axes (M, Q ou Q₁) sur au moins une des sections d'une surface de réglage (E, E₁) déterminée par la surface frontale (14 ou 42), lesquels axes sont déterminés par les axes embrochables (32, 32a) de la poulie intermédiaire (34).

2. Prothèse selon la revendication 1, **caractérisée en ce qu'**au moins l'un des axes embrochables (32) est disposé avec un écartement radial (q) par rapport à l'axe (Q) de la poulie intermédiaire (34).

3. Prothèse dont la poulie intermédiaire est utilisée dans un évidement (44), qui lui correspond à peu près, de l'une des sections voisines (14, 22), selon la revendication 1 ou 2, **caractérisée en ce que** la poulie intermédiaire (34) est utilisée avec un axe embrochable centré (32ₐ) dans l'une de ses formes correspondantes ainsi que de manière excentrique dans un évidement (44, 46) formé sur une surface de connexion (42) de la tête articulée (40) et que l'autre axe embrochable (32) appliqué de manière excentrique est adapté à la surface frontale (14), de la tige (12) parallèle par rapport à la surface de connexion qui détermine un plan de réglage (E₁) de la prothèse (10).

4. Prothèse selon la revendication 2 ou 3, **caractérisée en ce que** le diamètre (d) de la poulie intermédiaire (34) correspond environ à la moitié du diamètre (d₂) de la tête articulée (40) et que sa hauteur (n) correspond environ à la hauteur correspondante de son axe embrochable (32, 32a), moyennant quoi il est préférable que la hauteur (n) de la poulie intermédiaire (34) corresponde à environ un tiers de son diamètre (d) .

5. Prothèse selon la revendication 4, **caractérisée par** un diamètre de l'axe (C₁), le rapport de sa longueur par rapport au diamètre (d) de sa poulie intermédiaire (34) étant compris entre 1 :2,5 et 1 :3,0.

6. Prothèse selon une des revendications 2 à 5, **caractérisée en ce que** l'écartement radial (q) de l'axe embrochable (32), conçu de manière excentrique, par rapport à l'axe (Q) de la poulie intermédiaire (34) correspond à environ la moitié du diamètre de l'axe (c₁).

7. Prothèse selon l'une quelconque des revendications 1 à 6, **caractérisée en ce que** l'axe embrochable (32, 32a) comporte une zone d'ajustage serré (38) de forme annulaire, dont la hauteur (s) correspond de préférence à environ un dixième à trois dixièmes de la hauteur de l'axe (n).

8. Prothèse selon l'une quelconque des revendications 1 à 7, **caractérisée en ce que** l'axe embrochable (32, 32a) pour le trou foré cylindrique (16, 46) se raccourcit de façon conique depuis la poulie intermédiaire.

9. Prothèse selon l'une quelconque des revendications 1 à 8, **caractérisée en ce que** le diamètre de l'axe embrochable (32, 32a) mesure de 1 à 9 mm, de préférence de 3 à 7 mm.

10. Prothèse selon l'une quelconque des revendications 1 à 9, **caractérisée par** un alésage tangentiel (31) pour une vis tangentielle au niveau de l'alésage foré (16, 46).

11. Prothèse selon l'une quelconque des revendications 1 à 10, **caractérisée en ce que** la poulie intermédiaire (34) a un diamètre (d) de 10 à 35 mm, de préférence de 12 à 30 mm.

12. Prothèse selon l'une quelconque des revendications 1 à 11, **caractérisée en ce que** la poulie intermédiaire (34) a une épaisseur (n) de 2 à 8 mm, de préférence de 3 à 7 mm.

13. Prothèse selon l'une quelconque des revendications 1 à 12, **caractérisée en ce que** l'évidement plat (44) pour la poulie intermédiaire (34) dans la tête articulée (40) est déplacé à partir de son axe central (M₁) jusqu'à 10 mm, de préférence jusqu'à 8 mm.

14. Prothèse selon l'une quelconque des revendications 1 à 13, **caractérisée en ce que** la surface de la poulie (35) de la poulie intermédiaire (34) et / ou la surface de connexion (42) de la tête articulée (40) sont / est prévue(s) avec une échelle (36, 36a) adaptée au sens de rotation.

15. Utilisation des sections réglables (12, 40) et d'une poulie intermédiaire (34) sur une surface de réglage (E, E₁) selon au moins l'une quelconque des revendications 1 à 14 pour une prothèse d'articulation de coude, de genou ou de hanche ou pour une prothèse d'articulation de main ou de pied.

16. Utilisation de l'association des sections réglables (12, 40) et d'une poulie intermédiaire (34) sur une surface de réglage (E, E₁) selon au moins l'une quelconque des revendications 1 à 14 pour une prothèse des disques de la colonne vertébrale.
